Europäisches Patentamt

European Patent Office

Office européen des brevets

(11) Veröffentlichungsnummer: **0 202 569**

**A1**

(12) **EUROPÄISCHE PATENTANMELDUNG**

(21) Anmeldenummer: **86106407.9**

(22) Anmeldetag: **12.05.86**

(51) Int. Cl.⁴: **A 61 K 37/43**

(30) Priorität: **21.05.85 DE 3518252**

(43) Veröffentlichungstag der Anmeldung:
**26.11.86 Patentblatt 86/48**

(84) Benannte Vertragsstaaten:
**AT BE CH DE FR GB IT LI LU NL SE**

(71) Anmelder: **Oppermann, Dirck Dr. Med.**
**Sertorius-Ring 13**
**D-6500 Mainz-Finthen(DE)**

(72) Erfinder: **Oppermann, Dirck Dr. Med.**
**Sertorius-Ring 13**
**D-6500 Mainz-Finthen(DE)**

(74) Vertreter: **Henkel, Feiler, Hänzel & Partner**
**Möhlstrasse 37**
**D-8000 München 80(DE)**

(54) **Nasenspray zur Bekämpfung männlicher und weiblicher Infertilität.**

(57) Beschrieben wird ein in bestimmten Einheitsdosen intranasal zu applizierendes Nasenspray zur Behandlung von männlicher und weiblicher Infertilität bei idiopathischem hypogonadotropem Hypogonadismus und Kallmann-Syndrom, verzögerter Pubertät bei hypogonadotropem Hypogonadismus und Kallmann-Syndrom sowie verzögerter Pubertät bei konstitutioneller Entwicklungsverzögerung und anderen gutartigen Ursachen der verzögerten Pubertät, das als Wirkstoff LHRH enthält.

EP 0 202 569 A1

Croydon Printing Company Ltd.

Die Erfindung betrifft ein Mittel in Form eines Nasensprays zur Bekämpfung männlicher und weiblicher Infertilität bei idiopathischem hypogonadotropem Hypogonadismus und Kallmann-Syndrom, verzögerter Pubertät bei hypogonadotropem Hypogonadismus und Kallmann-Syndrom sowie verzögerter Pubertät bei konstitutioneller Entwicklungverzögerung und anderen gutartigen Ursachen der verzögerten Pubertät.

Es ist bekannt, daß sich männlicher hypogonadotroper Hypogonadismus (K. von Werder und T. Eversmann in "Deutsche medizinische Wochenschrift" 109, Nr. 11, 432 - 434, (1984)) und Infertilität bei hypothalamischer Amenorrhö ( G. Leyendecker, L. Wildt, M. Hansmann in J. Clin. Endocrinol. Metab. 51, 1214 (1980)) durch pulsatile Gabe von Gonadotropin- Releasing-Hormon (LHRH) erfolgreich therapieren lassen. Diese Therapie beruht auf der Erkenntnis, daß sich eine fehlende oder gestörte pulsatile LH-Sekretion durch pulsatile Applikation von LHRH (14 - 20 Tage bei der Frau, etwa 3 Monate beim Mann), nicht dagegen durch eine hochdosierte einmalige Gabe von LHRH, substituieren läßt. Auf diese Weise wird es möglich, eine normale Funktion der Gonaden zu erreichen.

Die pulsatile LHRH-Applikation erfolgt heutzutage mittels einer handelsüblichen, quarzgesteuerten peristaltischen Pumpe, die zu bestimmten Zeitintervallen (z. B. alle 90 bis 120 min) für eine bestimmte Zeit (z. B. für 1 Minute) in Gang gesetzt wird. Hierbei werden aus einem LHRH-Reservoir über einen Verweilkatheter etwa 5 - 20 µg LHRH intravenös oder subkutan appliziert. Die Pumpe nebst Zubehör wird vom Patienten während des Behandlungszeitraums am Körper getragen (vgl. Firmenschrift "Zyklomat" der Ferring Arzneimittel GmbH, Kiel).

Nachteilig am Einsatz einer Pumpe zur pulsierenden LHRH-Applikation ist, daß sie nur durch Spezialisten, nicht aber durch den Patienten selbst eingesetzt werden kann und darüber hinaus auch noch für den Patienten äußerst lästig ist.

Es wurde auch bereits versucht, Patienten mit idiopathischem hypogonadotropem Hypogonadismus oder verzögerter Pubertät (J. Happ und Mitarbeiter in "Int. J. Fertil. " 25(3), 247 - 255 (1980)) bzw. mit hypothalamischem Hypogonadismus und Kallmann-Syndrom * durch intranasale Applikation von LHRH zu therapieren. In ersterem Falle erfolgte eine 6- bis 8-malige nasale Gabe von 200 µg LHRH während 3 Monaten, in letzterem Falle nach einer subkutanen LHRH-Behandlung (mittels einer Pumpe der genannten Art täglich eine 8-malige intranasale Gabe von 200 µg LHRH alle 2 h. In ersterem Falle konnte praktisch kein oder allenfalls ein vorübergehender Testosteronanstieg, der noch nicht einmal den Mittelwert des Testosteronnormalbereichs von Erwachsenen erreichte, festgestellt werden. Insbesondere wird in "Int. J. Fertil."25(3), S. 247 darauf hingewiesen, daß durch die intranasale LHRH-Therapie keine signifikanten Einflüsse auf klinische Parameter festzustellen waren (linke Spalte, Zeilen 1 bis 3 von unten) bzw. daß die Behandlungsergebnisse bzgl. des Einflusses auf die Spermatogenese enttäuschend sind (rechte Spalte, Zeilen 15 bis 17 von unten). Aus der Arbeit von D. Klingmüller und H. U. Schweikert (vgl. Abstract 202 vom Deutschen Kongreß für Endokrinologie, März 1985) geht lediglich hervor, daß sich durch die nasale Applikation von LHRH nach vorherge-

* (D. Klingmüller und H.U.Schweikert "Abstract 202 vom Deutschen Kongreß für Endokrinologie" im März 1985) gangener pulsatierender LHRH-Applikation mittels einer Pumpe die Spermatogenese aufrechterhalten läßt. Der genannten Arbeit ist nicht zu entnehmen, daß intranasal appliziertes LHRH alleine bei Patienten mit hypothalamischen Hypogonadismus die Spermatogenese bewirkt bzw. in Gang setzt.

Es gibt auch bereits ein Nasenspray mit LHRH als Wirkstoff. Dieser Nasenspray wird zur Therapie des Hodenhochstandes verwendet (vgl. Wissenschaftliche Monographie "Kryptocur" der Hoechst Aktiengesellschaft, Frankfurt, April 1982). Das empfohlene Dosierschema besteht in einer dreimaligen intranasalen Applikation eines Sprühstosses mit jeweils 200 µg LHRH jeweils in das linke und rechte Nasenloch, d. h. von 6 x 200 µg LHRH zusammengezogen auf 3 Gaben. Ein meßbarer Anstieg der Testosteronkonzentration im Serum konnte nicht festgestellt werden.

Es wurde nun überraschenderweise und im Gegensatz zu den Ausführungen von J. Happ und Mitarbeitern in Int. J. Fertil. 25(3), 1980, S. 247, rechte Spalte, gefunden, daß eine intranasale Applikation von LHRH bei Einhaltung eines speziellen Dosierschemas selbst eine Spermatogenese bzw. Reifung des Eifollikels bewirkt.

Gegenstand der Erfindung ist demzufolge ein Nasenspray zur Behandlung der männlichen und weiblichen Infertilität bei idiopathischem hypogonadotropem Hypogonadismus und Kallmann-Syndrom, verzögerten Pubertät bei hypogonadotropem Hypogonadismus und Kallmann-Syndrom sowie verzögerten Pubertät bei konstitutioneller Entwicklungsverzögerung und anderen gutartigen Ursachen der verzögerten Pubertät, welches dadurch gekennzeichnet ist, daß es LHRH enthält und pro Sprühstoß 190 µg LHRH freigegeben werden.

4

Die ideale Dosierung besteht in einer 9-maligen täglichen intranasalen Applikation von 190 µg LHRH alle 110 min.

In anderen Worten gesagt, besteht die Erfindung in der Verwendung von LHRH in nasaler Applikationsform bei der Bekämpfung der genannten Zustände unter Einhaltung des angegebenen Dosierschemas.

Hierbei ist es jedoch von entscheidender Bedeutung, daß dem Patienten mit dem erfindungsgemäßen Nasenspray auch eine exakte und für jede Applikation gleiche Einheitsdosis für den Wirkstoff LHRH an die Hand gegeben wird, da die Dosierung selbst nicht dem Patienten überlassen bleiben darf. Eine solche Einheitsdosis muß der Arzneimittelhersteller bereitstellen.

So kann er beispielsweise über die Ausgestaltung und Dimensionierung des Ventils des Sprühbehälters, in dem das erfindungsgemäße Nasenspray untergebracht ist, dafür sorgen, daß vom ersten bis zum letzten Sprühstoß unter Berücksichtigung der jeweiligen Konzentration des Nasensprays an LHRH eine Menge Sprühbehälterinhalt, d. h. Nasenspray, abgegeben wird, die genau 190 µg LHRH enthält. Denkbar ist auch, in "Einwegsprays" eine exakt 190 µg LHRH enthaltende Nasenspraymenge unterzubringen, so daß bei der Benutzung dieses "Einwegsprays" der gesamte Behälterinhalt (mit 190 µg LHRH) auf einmal abgegeben wird.

Der Vorteil des erfindungsgemäßen Mittels liegt in der im Vergleich zur pulsatorischen LHRH-Applikation mittels einer Pumpe wesentlich einfacheren Gestaltung der Therapie durch den Arzt und Patienten selbst und nicht zuletzt auch darin, daß sie im Gegensatz zur Einpflanzung einer Zyklomatpumpe nicht weh tut. Zur Einleitung und Überwachung der Therapie bedarf es keiner Spezialisten mehr, sie kann vielmehr von jedem x-beliebigen Arzt durchgeführt werden. Die Applikation selbst erfolgt durch den Patienten, der allerdings derart motiviert sein muß, daß er das Dosierschema korrekt einhält. Der mit dem erfindungsgemäßen Mittel erzielbare Erfolg gilt auch gegenüber der Arbeit von D. Klingmüller und H. U. Schweikert, da ersteres sowohl zur Einleitung als auch Erhaltung der Spermatogenese geeignet ist, während letztere Behandlungsmethode in einer kombinierten pulsatorischen LHRH-Verabreichung zur Einleitung der Spermatogenese und einer intranasalen Verabreichung von LHRH zur Unterhaltung der Spermatogenese besteht. Die nachteiligen Begleiterscheinungen bei Anwendung einer peristaltischen Pumpe der beschriebenen Art sind bei letzterer Arbeit nicht beseitigt.

Mit dem Dosierschema des beschriebenen Handelsprodukts zur Behandlung von Hodenhochstand lassen sich die erfindungsgemäß möglichen Therapien nicht erfolgreich durchführen, was offensichtlich auf die andersartige Pulsamplitude und Pulsfrequenz der LHRH-Dosierung zurückzuführen ist (vgl. hierzu auch K. von Werder und T. Eversmann "Deutsche Medizinische Wochenschrift" 109(11), S. 432, linke Spalte (1984)).

Abgesehen von den genannten Therapiemöglichkeiten eignet sich das erfindungsgemäße Mittel auch noch als Diagnostikum zur probatorischen Kurzzeitbehandlung zum Beweis eines LHRH-Mangelzustandes des Hypothalamus und zum Beweis der intakten Sekretionsreserve der Hypophyse (bzgl. LH und FSH).

Die Dosierung zu diesem Zweck besteht in einer 6- bis 16-maligen täglichen intranasalen Applikation von 50 bis 300 µg LHRH alle 1 bis 3 h während 1 bis 30 Tagen, vorzugsweise in einer 9-maligen täglichen intranasalen Applikation von 190 µg LHRH alle 110 min während 10 Tagen.

Das erfindungsgemäße Mittel besteht insbesondere aus einem Nasenspray mit LHRH als Wirkstoff und einem für einschlägige Arzneimittel üblichen Träger. Gegebenenfalls kann das erfindungsgemäße Mittel auch noch andere, dem eigentlichen Therapiezweck dienende Wirkstoffe sowie übliche Hilfsstoffe, wie Konservierungsmittel und dergleichen, enthalten.

Die folgenden Beispiele sollen die Erfindung näher veranschaulichen.

Beispiel 1

Ein 29-jähriger Mann mit bekanntem Kallmann-Syndrom (=idiopathischer hypothalamischer Hypogonadismus mit komplettem Riechverlust, d. h. Mangel an LHRH als Krankheitsursache des Hypogonadismus) kam wegen Kinderwunsch in die Behandlung.

Die jahrelange Substitutionstherapie mit Testosteron-

-7-

spritzen wurde abgesetzt. Bei der Untersuchung zwei Wochen nach Absetzung der Testosteronspritzen war der körpereigene Testosteronspiegel mit 70 ng/dl erwartungsgemäß sehr niedrig (Normalwert des erwachsenen Mannes: 300 - 1200 ng/dl); die Samenuntersuchung zeigte keinerlei Spermien (=Azoospermie), wie üblich bei Kallmann- Syndrom.

Die Behandlung wurde mit einer intranasalen Gabe eines LHRH-Dosier-Aerosols mit 9 mal 190 µg LHRH alle 110 min durchgeführt. Nach zwei Wochen Behandlungsdauer stieg der Testosteronspiegel bis zum normal männlichen Bereich an. Nach dem 3. Behandlungsmonat konnten in der Samenanalyse 20 bis 40 Mio. Spermien pro Milliliter Samenflüssigkeit nachgewiesen werden (= unterer Normalbereich); das Hodenvolumen war beidseits von 7 ml auf 15 ml (Normwert des Mannes 12 bis 25 ml) angestiegen.

Ende des 5. Behandlungsmonats wurde die Frau des Patienten schwanger.

Die Behandlung wurde gut vertragen, es traten keine Nebenwirkungen auf, zur exakten Einhaltung des Dosierintervalles erwies sich eine normale Armbanduhr als völlig ausreichend.

Beispiel 2

Eine 23-jährige Frau mit primärer Amenorrhö (fehlende Periodenblutung, noch nie bisher aufgetreten) mit normalem Riechvermögen kam zur Behandlung der Amenorrhö in die Klinik.

Die körperliche gynäkologische Untersuchung war unauf- fällig.

Hormonell lag ein hypothalamischer Hypogonadismus mit

-8-

stark erniedrigten Werten von Östradiol, LH und FSH vor.

Die Behandlung wurde mit einer intranasalen Gabe eines LHRH-Dosier-Aerosols mit 9 mal 190 μg LHRH alle 110 min durchgeführt. Die Basaltemperatur (Aufwachtemperatur) wurde jeden Morgen gemessen. Täglich wurde eine Ultraschalluntersuchung der Eierstöcke durchgeführt, um die Reifung des Eifollikels zu beobachten.

Am 15. Behandlungstag konnte im Ultraschall ein sprungreifer Eifollikel von 2 cm Durchmesser im linken Eierstock nachgewiesen werden; am darauffolgenden Tag stieg die Basaltemperatur an und blieb für 13 Tage um 0,5°C erhöht, danach trat eine leichte Periodenblutung von 3 Tagen Dauer ein. Am 23. Tag der Behandlung war das Progesteron im Serum 19 ng/ml, eine Ovulation praktisch beweisend.

Die Behandlung wurde nebenwirkungsfrei gut vertragen. Die Untersuchungsergebnisse zeigen, daß durch die o. g. Behandlung ein Eisprung aufgetreten ist und somit eine Behandlung der Infertilität möglich ist.

LHRH = GnRH = Gonadorelin =
5-Oxo-L-prolyl-L-histidyl-L-tryptophyl-L-seryl-L-tyrosylglycyl-L-leucyl-L-arginyl-L-prolylglycinamid

Patentanspruch

Nasenspray zur Bekämpfung männlicher und weiblicher Infertilität bei idiopathischem hypogonadotropem Hypogonadismus und Kallmann-Syndrom, verzögerter Pubertät bei hypogonadotropem Hypogonadismus und Kallmann-Syndrom sowie verzögerter Pubertät bei konstitutioneller Entwicklungsverzögerung und anderen gutartigen Ursachen der verzögerten Pubertät, dadurch gekennzeichnet, daß es Gonadorelin (LHRH) enthält und pro Sprühstoß 190 µg LHRH freigegeben werden.

**Europäisches Patentamt**

## EUROPÄISCHER RECHERCHENBERICHT

| | EINSCHLÄGIGE DOKUMENTE | | EP 86106407.9 |
|---|---|---|---|

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | DE - A1 - 3 200 459 (HOECHST AG) <br><br> * Ansprüche; Seite 6, Zeilen 16-25; Seite 7, Zeile 10 * <br><br> -- | 1 | A 61 K 37/43 |
| A | EP - A2 - 0 136 781 (AMERICAN HOME PRODUCTS CORPORATION) <br><br> * Ansprüche; Seite 18, Zeilen 7-20 * <br><br> -- | 1 | |
| A | EP - A1 - 0 083 925 (HOECHST AKTIENGESELLSCHAFT) <br><br> * Ansprüche; Seite 5, Zeilen 24-36; Seite 7, Zeilen 4-21 * <br><br> ---- | 1 | |

| RECHERCHIERTE SACHGEBIETE (Int Cl 4) |
|---|
| A 61 K 37/00 |
| A 61 K 9/00 |

Der vorliegende Recherchenbericht wurde für alle Patentansprüche erstellt.

| Recherchenort | Abschlußdatum der Recherche | Prüfer |
|---|---|---|
| WIEN | 01-09-1986 | STÖCKLMAYER |